# FASCICULE DE BREVET EUROPEEN

(11) **EP 3 476 406 B1**
(45) Date de publication et mention de la délivrance du brevet: **08.12.2021**
(21) Numéro de dépôt: 18202892.8
(22) Date de dépôt: 26.10.2018
(51) Int. Cl.: G01N 33/22, A61L 2/28, C11D 3/48, C11D 3/40, C11D 11/00, C11D 3/386

(54) **KIT DE DÉTECTION DES CONTAMINATIONS RÉSIDUELLES SUR DES DISPOSITIFS MÉDICAUX**
KIT ZUR DETEKTION VON RESTKONTAMINATIONEN AUF MEDIZINISCHEN VORRICHTUNGEN
KIT FOR THE DETECTION OF RESIDUAL CONTAMINATION ON MEDICAL DEVICES

(30) Priorité: 27.10.2017 BE 201705778; 30.04.2018 BE 201800058
(43) Date de publication de la demande: 01.05.2019
(73) Titulaire: OneLIFE S.A., 1348 Louvain-La-Neuve (BE)
(72) Inventeur: Vanzieleghem, Thomas, 1348 Louvain-la-Neuve (BE); Fastrez, Sébastien, 7000 Mons (BE)
(74) Mandataire: Calysta NV

(56) Documents cités:
- EP-A1- 2 537 601
- EP-A1- 2 789 682
- WO-A1-2014/079938
- DE-A1-102006 062 379
- TOYONAGA M ET AL: "Protein detection e.g. on tableware surface - using dye soln. to assess cleaning properties of e.g. washing machine", WPI / 2017 CLARIVATE ANALYTICS,, vol. 1986, no. 36, 24 juillet 1986 (1986-07-24), XP002787408, -& JP S61 164158 A (ARUBOOSU YAKUSHIYOU KK) 24 juillet 1986 (1986-07-24)
- "Colorants acido-résistants pour les Mycobactéries", Pro-Lab diagnostics , novembre 2012 (2012-11), XP002789476, Extrait de l'Internet: URL:https://www.pro-lab.com/wp-content/upl oads/2016/11/Stains_TB_French.pdf [extrait le 2019-03-06]
- "Malachite green", Wikipedia , 3 janvier 2017 (2017-01-03), XP002789477, Extrait de l'Internet: URL:https://web.archive.org/web/2017010313 5649/https://en.wikipedia.org/wiki/Malachi te_green [extrait le 2019-03-06]
- "Rapid ethanol-based coomassie blue staining", INTERNET CITATION, 14 octobre 2017 (2017-10-14), XP002787409, Extrait de l'Internet: URL:https://web.archive.org/web/2017091207 3147/http://www.mundilab.com/protocolos/PA GE-destain.html [extrait le 2018-12-12]

## Description

La présente invention se rapporte à un kit de contrôle de la qualité du nettoyage de dispositifs médicaux.

Le retraitement de dispositifs médicaux consiste à appliquer des conditions de nettoyage selon des normes bien définies en Europe et au niveau mondial. Il permet d'éliminer les bactéries, les biofilms et les souillures organiques qui contaminent les dispositifs médicaux après usage. En effet, ceux-ci ont été en contact avec des patients, par exemple lors d'une opération chirurgicale ou lors d'une intervention chez le dentiste. Pour pouvoir réutiliser ces dispositifs médicaux en toute sécurité, il est primordial de pouvoir les nettoyer et stériliser ensuite efficacement afin de pouvoir les réutiliser pour d'autres interventions médicales.

Ces dispositifs médicaux présentent typiquement des parties appelées invasives et non invasives, lesquelles sont définies par rapport aux parties des dispositifs médicaux qui entrent en contact, respectivement, soit avec des matières organiques et/ou des composés protéiques et/ou des biofilms, soit avec des gants ou mains d'un praticien.

Il est vrai que les parties invasives localisées sur les dispositifs médicaux sont les plus susceptibles d'être directement en contact avec du sang ou des organes du patient. Ce sont donc les parties invasives des dispositifs médicaux qui nécessitent une attention toute particulière puisqu'il faut éviter que des traces de contamination organique ou biologique restent sur les dispositifs médicaux après stérilisation. Il est bien entendu que les parties non invasives des dispositifs médicaux doivent aussi être considérées puisqu'il n'est pas exclu qu'elles soient également contaminées lorsqu'elles sont en contact avec les parties invasives d'autres dispositifs médicaux.

Ainsi, le processus de stérilisation ne peut être garanti que si les dispositifs médicaux ne présentent plus de contamination organique détectable. Il est donc souhaité de stériliser du matériel qui soit suffisamment propre, en ce qu'il ne présente plus de composés protéiques et/ou de matières organiques et/ou de biofilms détectables.

Les contaminations qui se développent généralement dans les milieux hospitaliers sont un problème de santé publique que les professionnels de la santé souhaitent réduire voire, si possible, éradiquer. On connait les conséquences liées aux maladies de type Creutzfeldt Jakob ou encore les infections nosocomiales pour ne citer que celles-là. Dans la demande de brevet WO 2014079938 A1, il est décrit qu'un nettoyage de qualité des instruments médicaux agit de manière efficace sur les maladies nosocomiales. L'efficacité dudit procédé est mise en évidence par l'utilisation du kit décrit, dans la demande de brevet EP2537601 A1, une solution de coloration qui contient du bleu de Coomassie et une solution de nettoyage pour révéler de manière sélective les biofilms par vaporisation.

L'accumulation de composés protéiques et/ou de matières organiques et/ou de biofilms non éliminés sur des dispositifs médicaux qui subsistent donc même après stérilisation constitue une réelle problématique qu'il faut considérer pour pouvoir réduire les maladies qui se forment en milieu hospitalier. Il a aussi été constaté que la présence de composés protéiques sur les dispositifs médicaux diminue l'efficacité des stérilisations appliquées ultérieurement. De plus, la présence de ces composés protéiques augmente l'adhésion de bactéries comme *Staphylococcus aureus* qui peut conduire à des infections et maladies redoutables.

Or, il a été constaté que les techniques actuelles de stérilisation ne suffisent pas ou ne sont pas assez efficaces pour éliminer ces composés protéiques et/ou matières organiques et/ou de biofilms des dispositifs médicaux.

On connait du document JPS61164158, une composition pour détecter les protéines. Il divulgue de nombreux colorants, de nombreux solvants et régulateurs de pH qui peuvent être mélangés ensemble pour former cette composition. De multiple exemples sont donnés de compositions.

Malheureusement, le document décrit lui-même qu'il est compliqué de différencier l'amidon des protéines car il est possible que l'amidon soit lui aussi teinté par le colorant.

Pour cette raison, des techniques ont été récemment développées pour pouvoir contrôler la qualité du nettoyage des dispositifs médicaux avant et/ou après leur stérilisation. Cela a pour but de détecter des défauts de nettoyage, de pouvoir rectifier le tir en identifiant les dispositifs médicaux qui nécessitent un lavage additionnel et d'optimiser le processus de nettoyage sur une base objective. L'identification des dispositifs médicaux encore contaminés par la présence de composés protéiques et/ou de matières organiques et/ou de biofilms permet de les éliminer plus tard par application d'un nettoyage additionnel pour pouvoir les réutiliser ultérieurement, sans risque de contamination.

Il existe certaines techniques d'identification de composés protéiques et/ou de matières organiques et/ou de biofilms sur des dispositifs médicaux tels qu'un échantillonnage des protéines avec une solution de prélèvement et une quantification de celles-ci. Cette technique consiste à échantillonner les dispositifs médicaux avec une solution de décrochage de la contamination et de faire réagir les protéines présentes sur les dispositifs médicaux avec de l'o-phthaldialdéhyde/2-mercaptoéthanesulfonate de sodium pour révéler leur présence en laboratoire dans le but de pouvoir les quantifier (voir Smith et al. Journal of Foot and Ankle Research 2011, 4 :2).

Toutefois, ces techniques sont longues à appliquer et ne permettent pas de localiser les zones sur les dispositifs médicaux qui doivent encore être traités. Les méthodes connues sont couteuses, longues à appliquer et ne permettent pas d'analyser une série d'instruments médicaux en une fois.

Il existe aussi une technique ex situ qui consiste à utiliser un écouvillon qui est appliqué à la main sur la surface à contrôler. De cette façon, les protéines sont récoltées et le degré de contamination est indiqué au moyen d'un appareil dédié à la mesure de contamination.

Malheureusement, cette technique implique l'application de l'écouvillon pour chaque dispositif médical à contrôler, ce qui rend la tâche fastidieuse pour l'utilisateur qui devra avoir un écouvillon par instrument à analyser. De plus, la zone qui présente potentiellement une contamination n'est pas nécessairement visible ou accessible par l'opérateur qui peut passer à côté de la zone contaminée. Enfin, le taux de récupération de la souillure présente sur les instruments à l'aide de cet écouvillon est faible.

L'entreprise Synoptics Health propose également une machine de détection de protéines appelée « ProReveal » qui consiste à analyser des dispositifs médicaux un par un et face par face après pulvérisation d'un agent fluorescent non toxique. L'utilisateur place un seul instrument dans la machine qui contrôle après 3 minutes la qualité de nettoyage et permet aussi d'identifier par fluorescence la localisation des composés de protéines présents sur le dispositif médical analysé. L'analyse peut ainsi être poursuivie par introduction d'un autre instrument médical.

Malheureusement, cette méthode est couteuse, lente et peu pratique, notamment en ce qui concerne le nombre et la taille des instruments médicaux qui peuvent y être contrôlés.

Au vu de ce qui précède, il existe donc un réel besoin de fournir une méthode qui puisse permettre de vérifier la qualité du nettoyage appliqué sur des dispositifs médicaux avant et/ou après leur stérilisation en garantissant un contrôle de qualité rapide, pratique, fiable, reproductible, efficace et moins couteux.

La présente invention concerne plus particulièrement un kit de contrôle de la qualité de nettoyage d'un échantillon, qui comprend :
- une solution de coloration par trempage dudit échantillon, ladite solution de coloration comprenant un colorant dans une phase de dilution, et
- une solution de révélation par trempage dudit échantillon, ladite solution de révélation comprenant ladite phase de dilution.

Un tel kit a été décrit dans les exemples comparatifs de EP2537601 A1.

Malheureusement, selon ce document, il est recommandé d'utiliser plutôt une vaporisation des solutions de coloration et de nettoyage car les surfaces à traiter sont celles de l'industrie agroalimentaire. En effet, une seule face desdites surfaces est en contact avec une possible contamination et doit être nettoyée. De plus, le kit décrit dans la demande de brevet EP2537601 A1 est indiqué comme étant sélectif des biofilms, ne révélant pas la matière protéique (voir exemple comparatif 1 concernant le beurre et le lait).

Toutefois, en milieu hospitalier, les souillures susceptibles de transmettre des contaminations sont diverses et ne sont pas uniquement formées de biofilms.

L'invention a pour but de solutionner ce problème en procurant un kit qui permet la détection des souillures organiques, qu'elles soient formées de biofilms ou non.

il est prévu suivant l'invention de fournir un kit de contrôle de la qualité de nettoyage d'un échantillon comme indiqué précédemment, caractérisé en ce que ledit colorant dans ladite phase de dilution est compris dans le groupe des colorants constitué du Bleu de Coomassie et du noir Amide, ladite solution de coloration comprend un colorant à une concentration allant de 0.05% à 1%, ladite phase de dilution comprend au moins de l'acide citrique et/ou de l'acide lactique ; ladite phase de dilution est une phase de dilution compatible avec les exigences liées au milieu médical, duquel ledit échantillon provient, contenant un ou plusieurs dispositifs médicaux, éventuellement souillés de composés protéiques et/ou de matières organiques et/ou de biofilms présents sur lesdits dispositifs médicaux.

De manière étonnante, le kit selon l'invention permet de contrôler la qualité de nettoyage avant et/ou après stérilisation de manière très pratique étant donné qu'un échantillon contient une série de dispositifs médicaux qui peut être analysée en une fois et très rapidement, de l'ordre de quelques minutes, avec une fiabilité garantie et une grande capacité de détection.

En effet, à l'aide du kit selon l'invention, l'utilisateur désirant contrôler la qualité de nettoyage de dispositifs médicaux trempe d'abord l'échantillon dans la première solution de coloration afin que le colorant s'adsorbe sur les composés protéiques, et/ou les matières organiques et/ou les biofilms. Ensuite, l'échantillon coloré est trempé dans la deuxième solution de révélation pour éliminer le colorant libre (colorant excédentaire), en l'occurrence le colorant qui ne s'est pas adsorbé sur les composés protéiques et/ou matières organiques et/ou biofilms présents sur les dispositifs médicaux. Cette étape révèle ainsi les zones colorées qui devraient être à nouveau nettoyées et plus en profondeur de façon à complètement éliminer ces composés protéiques et/ou matières organiques et/ou biofilms de ces dispositifs.

Ainsi, lorsque rien n'est révélé par apparition d'une couleur correspondant au colorant utilisé, le contrôle est négatif par rapport au seuil de détection du kit selon l'invention, c'est-à-dire en ce qu'aucune trace de composés protéiques et/ou matières organiques (souillure) et /ou biofilms n'est révélée.

De plus, selon l'invention, la phase de dilution est compatible avec les exigences liées au milieu médical. Ladite phase de dilution est caractérisée par une volatilité réduite ce qui est très important. En effet, contrairement à l'industrie alimentaire qui comporte de grands halls, les espaces de laveries du milieu médical sont confinés et les équipes de nettoyage sont exposées aux vapeurs tout au long de la journée. Il faut donc pouvoir réduire les vapeurs qui peuvent être toxiques pour le personnel exposé.

Cela permet donc de fournir un kit de contrôle de nettoyage avant et/ou après stérilisation aisé à appliquer puisqu'il ne nécessite pas de dispositif additionnel couteux. L'utilisation d'un tel kit est simple à mettre en œuvre sur site étant donné que, en pratique, il suffit d'avoir un panier percé à immerger dans un premier contenant avec la solution de coloration et, ensuite, dans un deuxième contenant avec la solution de révélation, les premier et deuxième contenants étant de préférence munis d'un couvercle.

Le panier percé contenant l'échantillon est plongé dans la solution de coloration présente dans le premier contenant durant une période de temps inférieure à 10 minutes, de préférence inférieure à 7 minutes, plus préférentiellement allant de 2 à 5 minutes, encore plus préférentiellement égale à 5 minutes.

Ledit panier percé contenant l'échantillon est ensuite égoutté pendant environ 30 secondes au-dessus du premier contenant. Ensuite, ledit panier percé est amené au-dessus du deuxième contenant et est plongé dans la solution de révélation durant une période de temps inférieure à 5 minutes, de préférence inférieure à 3 minutes, plus préférentiellement allant de 0 à 2 minutes, encore plus préférentiellement égale à 2 minutes.

Ledit échantillon composé des dispositifs médicaux est ensuite récupéré pour une identification par coloration dans le visible.

L'échantillon constitué d'une série de dispositifs médicaux est donc plongé dans les premier et deuxième contenants au moyen d'un panier percé ce qui facilite le transfert d'un contenant à l'autre.

Ceci démontre bien la facilité de mise en oeuvre et le faible coût de l'utilisation d'un tel kit. De plus, il ne nécessite que des éléments qui équipent déjà typiquement les laveries.

En quelques minutes, l'utilisateur est en mesure d'identifier toutes les souillures formées sur les dispositifs médicaux analysés en temps très court, par rapport aux dispositifs existants.

Etant donné que ce kit révèle par identification par coloration dans le visible les zones affectées sur les dispositifs médicaux, il est par ailleurs aisé d'identifier les zones invasives et non invasives qui présentent des souillures. Cet aspect confère également un avantage pédagogique à la technologie qui permet de former et conscientiser les équipes de nettoyage des dispositifs médicaux.

Le kit selon l'invention est également avantageux en ce qu'il permet de mesurer l'efficacité de compositions détergentes et/ou enzymatiques qui ont pour but premier de nettoyer voire stériliser des dispositifs médicaux. En effet, en fonction du résultat obtenu par l'utilisation du kit selon l'invention, il est possible d'affiner la force ou l'efficacité de la composition de nettoyage en fonction des dispositifs médicaux à traiter.

De cette façon, il est possible de fournir différentes compositions de nettoyage de souillure dont l'efficacité peut être efficacement contrôlée, ce qui permet en finalité d'utiliser des détergents et/ou des enzymes à bon escient en fonction du nettoyage à réaliser et en fonction du type de dispositifs médicaux à traiter. Cela permet un ajustement des paramètres du protocole appliqué de manière objective en tenant compte (de la chimie) des détergents, du temps de contact, de la température et de l'action mécanique utilisée.

Le kit selon l'invention s'applique in situ, ce qui constitue un avantage certain par rapport aux techniques actuelles étant donné que la précision d'application sur l'ensemble de la surface à traiter est garantie à chaque contrôle de qualité, ce qui rend ce processus sûr, rapide et efficace pour l'utilisateur, en ce sens que la totalité de la surface des dispositifs médicaux est exposée au colorant du kit.

Le but de la présente invention est de fournir un kit de contrôle qualité qui peut être utilisé après nettoyage de dispositifs médicaux avant et/ou après leur stérilisation.

Au sens de la présente invention, on comprend par les termes « in situ », le fait d'immerger des dispositifs médicaux dans une solution, de préférence dans une zone où ils sont utilisés ou nettoyés et de détecter les contaminations potentielles directement sur la surface des dispositifs médicaux. Le contrôle indiqué au sens de la présente invention est réalisé en immergeant les dispositifs médicaux.

Au sens de la présente invention, le terme « comprendre » peut avantageusement être remplacé par le terme « constitué de » qui permet d'exclure la présence de tout autre élément défini par rapport au terme considéré.

Au sens de la présente invention, il faut entendre par le terme « stérilisation » tout moyen de stérilisation ou de désinfection de dispositifs médicaux, par exemple stérilisation vapeur, en autoclave, stérilisation à l'oxyde d'éthylène ou encore tout autre moyen qui a pour but d'éliminer complètement des microorganismes.

De plus, lorsqu'un dispositif médical est identifié comme possédant des contaminations résiduelles, le fait qu'elles apparaissent colorées facilite l'évaluation du nettoyage subséquent. En effet, si un dispositif médical apparait positif, il faut alors le refaire passer en nettoyage. Comme la souillure est colorée, l'opérateur peut ainsi directement identifier si le deuxième nettoyage est suffisant. S'il reste des traces colorées, le nettoyage est insuffisant. S'il n'y a plus de traces colorées et donc plus d'agent de coloration, le nettoyage est efficace. Un procédé efficace de nettoyage est, par exemple, de disposer les dispositifs médicaux souillés et donc colorés dans un bain à ultrasons contenant une composition détergente contenant une ou plusieurs enzymes (protéases, laccases, lipases, amylase, DNase, cellulases, dispersine).

De préférence, ladite solution de coloration comprend un colorant à une concentration allant de 0.05% à 1%, de préférence à une concentration allant de 0.05% à 0.5% et plus préférentiellement à une concentration allant de 0.1% à 0.2%.

Plus préférentiellement, ladite solution de coloration présente un pH compris entre 1 et 9, de préférence entre 1 et 7, plus préférentiellement entre 1 et 5, encore plus particulièrement entre 1 et 3 ou bien entre 3 et 5.

Encore plus préférentiellement, ladite phase de dilution est une solution aqueuse.

Avantageusement, ladite phase de dilution comprend au moins un acide et de l'eau.

Plus avantageusement, ladite phase de dilution comprend en outre au moins un solvant organique. De manière particulièrement avantageuse, ladite phase de dilution comprend au moins un acide choisi dans le groupe constitué de l'acide lactique, de l'acide citrique, de l'acide acétique, de l'acide oxalique, de l'acide phosphorique et éventuellement au moins un solvant organique choisi dans le groupe constitué de l'éthanol, de l'isopropanol, des éthers de glycol, de l'éther, de l'acétone, de propanol, de butanol, des éthers de pétrole et de leurs mélanges.

De manière encore plus avantageuse, ladite phase de dilution comprend au moins de l'acide citrique et/ou de l'acide lactique.

Avantageusement, ladite phase de dilution comprend au moins un additif et de l'eau, par exemple ledit additif peut être un sel inorganique et/ou un conservateur antimicrobien choisi dans le groupe constitué du sulfate d'ammonium, du chlorure de sodium, sorbate de potassium, methylisothiazoline et de sulfite et de leurs mélanges.

La liste de solvants organiques, acides et additifs énumérée ci-avant n'est pas exhaustive et peut comprendre d'autres acides, solvants ou additifs convenant pour la formation de la phase de dilution compatible avec les exigences du milieu médical.

Selon un mode préféré du kit selon l'invention, lesdits dispositifs médicaux sont choisis dans le groupe constitué d'instruments de dentisterie, chirurgicaux avec ou sans lumens, endoscopes et tout autre dispositif médical, lesdits dispositifs médicaux étant formés de matériaux de faible porosité et/ou de faible rugosité. En particulier, les matériaux utilisés dans les dispositifs médicaux et dans le domaine médical sont généralement l'acier inoxydable, le titane, le carbure de tungstène, les matériaux chromés, les plastiques, le verre, l'aluminium, l'aluminium anodisé, acier inox, cuivre, laiton, matières synthétiques à base de polyamide, poly-éthylène, PVC, POM, l'ABS, le verre acrylique, le polyphénylsulfone, le polypropylène, le téflon (PTEE), les polycarbonates, dans leurs formes compatibles avec les applications médicales et leur combinaisons. Les dispositifs médicaux formés de caoutchouc, élastomères, latex, céramiques, de bois, de surfaces naturellement fortement colorées et/ou leur mélanges sont exclus à cause de leur porosité et/ou rugosité.

Selon un mode particulier, le kit selon l'invention présente une limite de détection des protéines inférieure à 100 µg/cm², de préférence inférieure à 75 µg/cm²

D'autres formes de réalisation du kit suivant l'invention sont indiquées dans les revendications annexées.

La présente invention se rapporte également à un procédé de contrôle de la qualité de nettoyage des dispositifs médicaux.

Il existe comme discuté précédemment des procédés de contrôle de la qualité du nettoyage des dispositifs médicaux.

Pour résoudre le problème, il est prévu selon l'invention de fournir un procédé de contrôle de la qualité du nettoyage des dispositifs médicaux comprenant les étapes suivantes :
- une mise à disposition d'un échantillon prélevé aléatoirement parmi des dispositifs médicaux, préalablement nettoyé en vue d'une stérilisation, ou bien préalablement nettoyé et stérilisé en vue d'un contrôle qualité.
- une mise en contact dudit échantillon par une solution de coloration qui comprend un colorant dans une phase de dilution compatible les exigences liées au milieu médical,
- contrôle de la qualité de nettoyage de l'échantillon par une éventuelle identification par coloration.

Un tel procédé est déjà connu de la société Synoptics Health et a été décrit précédemment dans ce document. Ledit procédé se nomme « ProReveal Protein Détection Test ».

Malheureusement, si ce procédé est très efficace en terme de qualité de détection des protéines, sa mise en œuvre se révèle nettement plus compliquée. D'une part, elle nécessite l'installation couteuse d'une machine spécifique et donc de dispositifs additionnels. De plus, l'analyse est lente car chaque face de chaque dispositif médical doit être analysée une par une et ce pour chaque dispositif médical séparément.

Chaque dispositif médical est introduit dans la machine après avoir été vaporisé sur une face par la solution de coloration fluorescente. Ladite machine ne peut analyser qu'une seule face d'un dispositif à la fois. Il faut retourner, ensuite, le dispositif médical, le vaporiser de nouveau et le remettre dans la machine pour permettre que l'analyse du dispositif médical soit complète. De plus il existe toujours un risque qu'avec la vaporisation, la face du dispositif médical qui sera analysée ne soit pas totalement recouverte de la solution de coloration fluorescente.

L'analyse effectuée par la machine est obligatoire pour permettre l'identification par fluorescence des composés protéiques.

Ensuite, ce type de procédés existants nécessite une adaptation des procédés de lavage existants, ce qui perturbe intégralement le flux logistique en place. L'adaptation des flux logistiques en place a pour résultat que l'étape de contrôle est souvent négligée, prenant du temps. Le milieu médical a alors tendance à parfois s'assoir sur ses certitudes ou sur les tests réalisés en amont par les départements de contrôle qualité au moment de la définition du flux logistique à implémenter pour le nettoyage et la stérilisation, sans effectuer de contrôle régulier sur la qualité du nettoyage. Pourtant, il est manifeste que même après nettoyage, des parties de dispositifs médicaux portent encore des traces de souillures organiques, lesquelles peuvent être source de contamination au patient, même après stérilisation, en particulier lorsque les souillures organiques contiennent du biofilm.

Il existe donc un besoin de pouvoir adapter un procédé de contrôle de la qualité du nettoyage dans l'environnement contraint des laveries du milieu médical.

L'invention a pour but de solutionner le problème en procurant un procédé pour contrôler la qualité de nettoyage d'un échantillon efficace, rapide et facile à reproduire et qui est adaptable dans les laveries du milieu médical ou hospitalier.

A cette fin, le procédé selon l'invention est caractérisé en ce que ledit colorant dans ladite phase de dilution est compris dans le groupe des colorants constitué du Bleu de Coomassie et du Noir Amide, ledit échantillon comprend un ou plusieurs dispositifs médicaux de faible porosité et/ou de faible rugosité choisis aléatoirement et est posé dans un panier percé, ladite mise en contact étant effectuée par un premier trempage par immersion dudit panier percé contenant l'échantillon dans une solution de coloration et par un deuxième trempage après coloration, par immersion dudit panier percé contenant l'échantillon dans une solution de révélation comprenant ladite phase de dilution et en ce que ladite identification par coloration est une coloration dans le visible de composés protéiques et/ou de matières organiques et/ou biofilms.

De manière étonnante, le procédé selon l'invention, bien qu'étant extrêmement simple à mettre en œuvre dans le milieu médical ou hospitalier permet de contrôler la qualité de nettoyage avant et/ou après stérilisation de manière très pratique. Il permet, avec une limite de détection très faible et donc un niveau de sensibilité élevé, de détecter une contamination résiduelle sur divers dispositifs médicaux qui, ensemble, forment l'échantillon, même dans des zones inaccessibles. Cela entraine un gain de temps plus que considérable qui est très important dans le milieu médical ou hospitalier et, cela en utilisant des équipements typiquement présents dans le milieu médical ou hospitalier tels que des paniers percés et des bacs de trempage. Les dispositifs testés sont plus nombreux et permettent une meilleure représentation de l'échantillonnage contaminé.

Dans le procédé selon l'invention, les dispositifs médicaux posés dans le panier percé sont plongés dans le premier bac de trempage comprenant la solution de coloration. Par la suite, lesdits dispositifs médicaux sont plongés, après égouttage de quelques secondes, dans le second bac de trempage comprenant la solution de révélation. Lesdits dispositifs médicaux sont ensuite identifiés par coloration dans le visible. Ces étapes de trempage permettent une répartition uniforme et sur toutes les faces de chaque dispositif médical de la solution de coloration et y compris dans les tubes.

De plus, selon l'invention, la phase de dilution est compatible avec les exigences liées au milieu médical. Ladite phase de dilution est caractérisée par une volatilité réduite ce qui est très important. En effet, les espaces de laveries associés au milieu médical sont confinés et les équipes de nettoyage sont exposées aux vapeurs tout au long de la journée. Par conséquent, le procédé selon la présente invention permet une mise en œuvre aisée dans un milieu très contraignant en alliant efficacité et rapidité de détection tout en assurant une compatibilité avec les contraintes en mettant en œuvre une solution de coloration dont le colorant et la phase de dilution sont efficaces mais peu toxiques ou agressifs pour les utilisateurs. Ledit colorant et ladite phase de dilution facilitent aussi le nettoyage subséquent car les traces de souillure restent colorées jusqu'à la disparition de la souillure contrairement à la solution utilisée avec la machine « ProReveal » qui nécessite de retester la propreté avant la stérilisation.

Enfin, l'identification n'est pas limitée à l'identification exclusive des composés protéiques mais inclut aussi les souillures organiques et/ou de biofilms.

De préférence, ledit premier trempage par immersion dudit panier percé contenant l'échantillon est réalisé durant une période de temps inférieure à 10 minutes, de préférence inférieure à 7 minutes, plus préférentiellement allant de 2 à 5 minutes, encore plus préférentiellement égale à 5 minutes.

Plus préférentiellement, ledit deuxième trempage par immersion dudit panier percé contenant l'échantillon est réalisé durant une période de temps inférieure à 5 minutes, de préférence inférieure à 3 minutes, plus préférentiellement allant de 0 à 2 minutes, encore plus préférentiellement égale à 2 minutes.

De manière avantageuse, lesdits premier et deuxième trempages par immersion dudit panier percé contenant l'échantillon consistent à immerger complètement lesdits dispositifs médicaux dans les solutions susvisées.

Selon un mode préféré comprenant, en outre, une étape de rinçage, de préférence à l'eau, dudit échantillon, appliqué directement après ledit deuxième trempage, par immersion dudit panier percé contenant l'échantillon.

D'autres formes de réalisation du procédé suivant l'invention sont indiquées dans les revendications annexées.

La présente invention se rapporte également à une utilisation du kit selon la présente invention pour contrôler la qualité de nettoyage de dispositifs médicaux avant et/ou après stérilisation.

D'autres formes de réalisation de l'utilisation suivant l'invention sont indiquées dans les revendications annexées.

La présente invention se rapporte en outre à l'utilisation du kit selon la présente invention pour ajuster l'efficacité de nettoyage d'une composition détergente comprenant de préférence une enzyme.

D'autres formes de réalisation de cette utilisation suivant l'invention sont indiquées dans les revendications annexées.

D'autres caractéristiques, détails et avantages de l'invention ressortiront de la description donnée ci-après, à titre non limitatif et en faisant référence exemples annexés.

Le colorant selon l'invention est un colorant choisi dans un groupe spécifique et restreint comme par exemple le bleu de Coomassie, le rouge Nil, le noir Amide 10B, le vert de Malachite et peut être de couleur, noire, rouge, pourpre, vert, mauve, etc. mais ne peut pas être le bleu de méthylène, la fuchsine, le Crystal violet et le rouge Congo. En effet, le groupe restreint a été identifié pour sa spécificité aux protéines/polypeptides présents dans les souillures organiques et les biofilms contaminant les dispositifs médicaux.

En fonction du type de colorant utilisé, le type de solvant sera donc choisi de manière à permettre, d'une part, la solubilisation du colorant et d'autre part à être compatible avec les exigences liées au milieu médical et aux espaces contraints des laveries.

Il a été constaté que la phase de dilution telle que celle du document EP2537601 A1 constituée d'acide acétique, d'eau et d'éthanol et d'isopropanol dégage une odeur peu agréable et extrêmement irritante pour le personnel des laveries.

Dans le cadre de l'application médicale visée dans le cadre selon la présente invention, il est préférable de fournir une phase de dilution qui ne dégage pas d'odeur désagréable.

Pour ce faire, il est par exemple possible, en fonction du colorant, de former la phase de dilution suivant l'invention en mélangeant de l'eau et au moins un additif ou en mélangeant de l'acide citrique et/ ou de l'acide lactique ou de former la phase de dilution en mélangeant de l'eau, de l'éthanol éventuellement en présence d'isopropanol et d'acide citrique et/ou acide lactique pour réduire voire éliminer cette odeur désagréable pour l'utilisateur.

Il est bien entendu que tout autre phase de dilution peut être formée dans le cadre de la présente invention en préparant cette phase de dilution avec d'autres composés qui permettent aussi de fournir une phase de dilution neutre à l'odorat.

Comme on peut le constater, la phase de dilution selon l'invention est une phase compatible avec les exigences du milieu médical.

Dans le cadre de la présente invention, il est également possible d'avoir une seule étape de révélation et de rinçage par l'utilisation d'un colorant hydrosoluble qui permettrait de ne plus avoir une étape additionnelle de rinçage éventuelle.

### Exemple 1 :

Du lait écrémé est dilué avec de l'eau déminéralisée (50 fois, 200 fois et 300 fois). Chaque goutte de chaque dilution de lait écrémé est déposée sur des coupons en inox. Lesdits coupons sont, dans un premier temps, séchés pendant 1 heure à 60°C et , dans un deuxième temps, séchés pendant 1 heure à 130°C. Ces étapes de séchage ont pour objectif de fixer les protéines sur lesdits coupons.

La moitié des coupons est plongée pendant 5 minutes dans une solution de coloration, appelée « solution de coloration 1 », contenant pour 100 ml, 0.1 g de Bleu de Coomassie dans une phase de dilution comprenant environ 45 g d'eau adoucie, 40 g d'éthanol, 10 g d'isopropanol, 1 g d'acide acétique 80%, 4 g d'acide citrique.

La moitié des coupons est ensuite plongée pendant 2 minutes dans la « solution de révélation 1 » constituée de la même phase de dilution que la solution de coloration 1. Les coupons sont ensuite séchés à l'air libre.

L'autre moitié des coupons est plongée pendant 5 minutes dans une solution de coloration, appelée « solution de coloration 2 », contenant pour 100 ml, 0.2g de Noir Amide dans une phase de dilution comprenant environ 98 g d'eau adoucie et 2 g d'acide citrique.

La moitié des coupons est ensuite plongée pendant 2 minutes dans la « solution de révélation 2 » constituée approximativement de la même phase de dilution que la solution de coloration 2. Les coupons sont ensuite séchés à l'air libre.

Les résultats de l'exemple 1 montrent des résultats très satisfaisants concernant la pénétration du colorant Noir Amide et le seuil de sensibilité. Le Noir Amide pénètre avec une intensité satisfaisante les coupons aux différentes dilutions testées 50, 200 et 300 fois par rapport au contrôle négatif. De plus, les résultats montrent que la phase de dilution n'est pas dépendante de solvants organiques et d'acide acétique pour obtenir un seuil de sensibilité élevé. De plus, ne pas utiliser de solvants organiques ni d'acide acétique a permis d'éliminer les vapeurs des solvants organiques et de l'acide acétique qui sont irritants et nocifs pour le personnel.

### Exemple 2

Du lait écrémé est dilué avec de l'eau déminéralisée (50 fois, 200 fois). Chaque goutte de chaque dilution de lait écrémé est déposée sur des coupons en inox. Lesdits coupons sont, dans un premier temps, séchés pendant 1 heure à 60°C et , dans un deuxième temps, séchés pendant 1 heure à 130°C. Ces étapes de séchage ont pour objectif de fixer les protéines sur lesdits coupons.

La moitié des coupons est plongée pendant 5 minutes dans une solution, appelée « solution 1 », contenant pour 100 ml, 0.1 g de Bleu de Coomassie dans une phase de dilution comprenant environ 70 g d'eau adoucie, 15 g d'éthanol, 10 g d'isopropanol, 5 g d'acide lactique.

La moitié des coupons est ensuite plongée pendant 2 minutes dans la solution de révélation 1 constituée approximativement de la même phase de dilution que la solution de coloration 1. Les coupons sont ensuite séchés à l'air libre.

L'autre moitié des coupons est plongée dans une solution, appelée « solution 2 », contenant pour 100 g, 0.1 g de Bleu de Coomassie dans une phase de dilution contenant environ 70 g d'eau adoucie, 15 g d'éthanol, 10 g d'isopropanol, 1 g d'acide acétique, 4 g d'acide citrique.

La moitié des coupons est ensuite plongée pendant 2 minutes dans la solution de révélation 1 constituée approximativement de la même phase de dilution que la solution de coloration 1. Les coupons sont ensuite séchés à l'air libre.

Les résultats de l'exemple 2 montrent que la diminution d'éthanol de 40 g à 15 g ne réduit pas l'efficacité. De plus, le remplacement du mélange acide citrique/ acide acétique par de l'acide lactique ne modifie pas la pénétration du colorant ni le seuil de sensibilité de détection. Le Bleu de Coomassie pénètre avec toujours avec une intensité satisfaisante les coupons aux différentes dilutions testées 50 et 200 fois par rapport au contrôle négatif lorsque la phase de dilution ne contient que 15 g d'éthanol et lorsque de l'acide lactique est utilisée.

### Exemple 3

Une solution de coloration est préparée en mélangeant 1 g de bleu de Coomassie dans une phase de dilution constituée de 450 g d'eau, 400 g d'éthanol, 100 g d'isopropanol, 40 g d'acide citrique et 10 g d'acide acétique. Une solution de coloration 5 litres est préparée en tenant compte des quantités précitées multipliées par 5.

Une solution de révélation est préparée avec les mêmes éléments que ceux repris pour former la phase de dilution susvisée, excepté qu'un volume de 5 litres est préparé en tenant compte des mêmes rapports que ceux susvisés pour former la phase de dilution. La solution de révélation ne contient pas le bleu de Coomassie.

5 litres de la solution de coloration sont versés dans un premier bac et 5 litres de la solution de révélation sont versés dans un deuxième bac.

Un panier percé contenant un échantillon constitué de 76 instruments de dentisterie, parmi lesquels on retrouve 25 pinces, 46 forceps et 5 pinces de microchirurgie.

Ce panier percé contenant l'échantillon d'instruments de dentisterie est complètement immergé dans le premier bac contenant la solution de coloration. Le bac est fermé par un couvercle et le panier est laissé dans ce bac durant 5 minutes pour colorer tous les instruments de dentisterie.

Ensuite, après ouverture du premier bac par retrait de son couvercle, le panier contenant l'échantillon coloré est complètement immergé dans le deuxième bac contenant la solution de révélation. Ce deuxième bac est à son tour fermé par un autre couvercle et le panier est laissé durant 2 minutes pour retirer l'excédent de colorant. Cela permet de révéler les parties des instruments de dentisterie souillées par les composés protéiques et/ou les matières organiques et/ou biofilm.

Ainsi, une couleur bleu visible à l'œil nue permet d'identifier les zones souillées sur chaque instrument contrôlé par le kit selon l'invention.

Ce test a permis de révéler que 84 % des instruments de dentisterie contrôlés contenaient des souillures alors qu'ils étaient issus du lavage et prêts à être stérilisés.

Cela a permis d'appliquer une étape ultérieure de stérilisation/lavage afin de complètement éliminer les souilles identifiées.

Le matériel ainsi traité a ainsi pu réintégrer une ligne constituée d'autres matériels chirurgicaux prêt à l'emploi.

De manière préférentielle, il est également possible d'ajouter un troisième bac contenant de l'eau afin d'éliminer encore quelques résidus.

### Exemple Comparatif 1 :

Comme dans l'exemple 3, une solution de coloration est préparée en mélangeant 1 g de bleu de Coomassie dans une phase de dilution constituée de 450 g d'eau, 400 g d'éthanol, 100 g d'isopropanol, 40 g d'acide citrique et 10 g d'acide acétique. Une solution de coloration 1 litre est préparée en tenant compte des quantités précitées.

Une solution de révélation est préparée avec les mêmes éléments que ceux repris pour former la phase de dilution susvisée. La solution de révélation ne contient pas le bleu de Coomassie.1 litre de la solution de coloration est versé dans un premier vaporisateur et 1 litre de la solution de révélation est versé dans un deuxième vaporisateur.

30 instruments de dentisterie sont déposés dans un évier, parmi lesquels on retrouve 10 pinces, 10 forceps et 10 pinces de microchirurgie.

L'échantillon d'instruments de dentisterie est vaporisé à l'aide du premier vaporisateur contenant la solution de coloration. L'échantillon des instruments de dentisterie est laissé à l'air libre durant 5 minutes jusqu'à coloration de tous les instruments de dentisterie.

Ensuite, l'échantillon coloré est vaporisé à l'aide du second vaporisateur contenant la solution de révélation. L'échantillon est laissé à l'air libre durant 2 minutes pour retirer l'excédent de colorant. Cela permet de révéler les parties des instruments de dentisterie souillées par les composés protéiques et/ou les matières organiques et/ou biofilms.

Ainsi, une couleur bleu visible à l'œil nue permet d'identifier les zones souillées sur chaque instrument contrôlé.

L'analyse visuelle des instruments permet de constater que l'exposition totale du matériel à évaluer n'est pas assurée. De plus, les parties des instruments présentant des géométries complexes ne sont pas exposées correctement au colorant et le risque de résultats faux-négatifs a été détecté au niveau des charnières des forceps. En effet, les instruments doivent être manipulées et retournés pour une vaporisation sur toutes les faces des instruments. Le personnel est exposé aux aérosols via les voies respiratoires et la vaporisation mène à une contamination des surfaces ouvertes.

Ce test permet donc de constater que l'immersion par trempage des instruments dans les solutions de coloration et de révélation permet une exposition totale de chaque face des instruments, une absence d'aérosols des solutions de coloration et de révélation contenant des solvants organiques et acides dans la phase de dilution et l'immersion par trempage permet un gain de temps non négligeable du nettoyage des surfaces ouvertes puisque les solutions de coloration et de révélation sont contenues dans des bacs de trempage.

La vaporisation comme technique de contrôle de la qualité de nettoyage est donc écartée.

De manière préférentielle, la forme de réalisation principale de la présente invention est une étape de contrôle aléatoire de la qualité de nettoyage de dispositifs médicaux sortants de l'étape de nettoyage avant stérilisation et/ou sortants de l'étape de stérilisation.

Ladite étape de contrôle aléatoire de la qualité de nettoyage est intégrée dans le flux logistique des laveries. Elle consiste à prélever un échantillon sur un nombre déterminé de paniers après nettoyage ou stérilisation. Le nombre déterminé de paniers dépend de la capacité de la laverie.

Un échantillon est composé d'un panier contenant un ou plusieurs dispositifs médicaux préalablement nettoyés et/ou préalablement stérilisés. Ledit échantillon est choisi au hasard et testé avec le kit de détection de contamination résiduelle.

Si un dispositif médical apparait encore souillé, la souillure est colorée et ceci permet de voir à l'œil nu où il y a lieu d'insister pendant le nettoyage pour éliminer la souillure.

Il est difficile de pouvoir mettre en place un contrôle qualité de tous les dispositifs médicaux pour les grandes structures lavant des milliers de dispositifs médicaux par jour. En effet, même si les temps de trempage sont très courts, si le contrôle qualité est effectué pour chaque dispositif médical, cela induira une diminution non négligeable du nombre de dispositifs nettoyés et/ou stérilisés par jour.

Par contre, l'invention prévoit aussi pour les petites structures, comme par exemple les dentisteries, que le prélèvement aléatoire est systématique. Le procédé de détection des contaminations résiduelles par utilisation du kit décrit dans la présente invention est alors mis en œuvre après chaque nettoyage.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Kit de contrôle de la qualité de nettoyage d'un échantillon, qui comprend :
- une solution de coloration par trempage dudit échantillon, ladite solution de coloration comprenant un colorant dans une phase de dilution, et
- une solution de révélation par trempage dudit échantillon, ladite solution de révélation comprenant ladite phase de dilution.
**caractérisé en ce que** ledit colorant dans ladite phase de dilution est compris dans le groupe des colorants constitué du bleu de Coomassie,
et du noir Amide, ladite solution de coloration comprend un colorant à une concentration allant de 0.05% à 1%, ladite phase de dilution comprend au moins de l'acide citrique et/ou de l'acide lactique ; ladite phase de dilution est une phase de dilution compatible avec les exigences liées au milieu médical, duquel ledit échantillon provient, contenant un ou plusieurs dispositifs médicaux, éventuellement souillés de composés protéiques et/ou de matières organiques et/ou de biofilms présents sur lesdits dispositifs médicaux.

2. Kit selon la revendication 1, dans lequel ladite solution de coloration comprend un colorant de préférence à une concentration allant de 0.05% à 0.5% et plus préférentiellement à une concentration allant de 0.1% à 0.2%.

3. Kit selon la revendication 1 ou 2, dans lequel ladite solution de coloration présente un pH compris entre 1 et 9, de préférence entre 1 et 7, plus préférentiellement entre 1 et 5, encore plus particulièrement entre 1 et 3 ou bien entre 3 et 5.

4. Kit selon la revendication l'une quelconque des revendications précédentes, dans lequel ladite phase de dilution est une solution aqueuse.

5. Kit selon l'une quelconque des revendications précédentes, dans lequel ladite phase de dilution comprend au moins un acide et de l'eau.

6. Kit selon l'une quelconque des revendications précédentes, dans lequel ladite phase de dilution comprend en outre un solvant organique.

7. Kit selon l'une quelconque des revendications précédentes, dans lequel ladite phase de dilution comprend au moins un acide choisi dans le groupe constitué de l'acide lactique, de l'acide citrique, de l'acide acétique, de l'acide oxalique, de l'acide phosphorique et éventuellement au moins un solvant organique choisi dans le groupe constitué de l'éthanol, de l'isopropanol, des éthers de glycol, de l'éther, de l'acétone, de propanol, de butanol, des éthers de pétrole et de leurs mélanges.

8. Kit selon l'une quelconque des revendications précédentes, dans lequel ladite phase de dilution comprend au moins un additif et de l'eau.

9. Kit selon l'une quelconque des revendications précédentes, dans lequel lesdits dispositifs médicaux sont choisis dans le groupe constitué d'instruments de dentisterie, chirurgicaux avec ou sans lumens, endoscopes et tout autre dispositif médical, lesdits dispositifs médicaux étant de faible porosité et/ou de faible rugosité sont formés d'au moins un matériau comme l'acier inoxydable, le titane, le carbure de tungstène, les matériaux chromés, les plastiques, le verre, l'aluminium, l'aluminium anodisé, acier inox, cuivre, laiton, matières synthétiques à base de polyamide, polyéthylène, PVC, POM, l'ABS, le verre acrylique, le polyphénylsulfone, le polypropylène, le teflon (PTFE), les polycarbonates, dans leur formes compatibles avec les applications médicales et leurs combinaisons et lesquels ont préalablement été soumis à un nettoyage en vue d'une stérilisation.

10. Procédé de contrôle de la qualité du nettoyage des dispositifs médicaux, de préférence par utilisation du kit selon l'une quelconque des revendications précédentes, comprenant les étapes suivantes :
- une mise à disposition d'un échantillon prélevé aléatoirement parmi des dispositifs médicaux, préalablement nettoyé en vue d'une stérilisation, ou bien préalablement nettoyé et stérilisé en vue d'un contrôle qualité.
- une mise en contact dudit échantillon par une solution de coloration qui comprend un colorant dans une phase de dilution compatible avec les exigences liées au milieu médical,
- contrôle de la qualité de nettoyage de l'échantillon par une éventuelle identification par coloration.
**caractérisé en ce que** ledit colorant dans ladite phase de dilution est compris dans le groupe des colorants constitué du Bleu de Coomassie et du Noir Amide, ledit échantillon comprend un ou plusieurs dispositifs médicaux de faible porosité et/ou de faible rugosité choisis aléatoirement et est posé dans un panier percé, ladite mise en contact étant effectuée par un premier trempage par immersion dudit panier percé contenant l'échantillon dans une solution de coloration et par un deuxième trempage après coloration, par immersion dudit panier percé contenant l'échantillon dans une solution de révélation comprenant ladite phase de dilution et **en ce que** ladite identification par coloration est une coloration dans le visible de composés protéiques et/ou de matières organiques et/ou biofilm.

11. Procédé selon la revendication 10, dans lequel ledit premier trempage par immersion dudit panier percé contenant l'échantillon est réalisé durant une période de temps inférieure à 10 minutes, de préférence inférieure à 7 minutes, plus préférentiellement allant de 2 à 5 minutes, encore plus préférentiellement égale à 5 minutes.

12. Procédé selon la revendication 10 ou 11, dans lequel ledit deuxième trempage par immersion dudit panier percé contenant l'échantillon est réalisé durant une période de temps inférieure à 5 minutes, de préférence inférieure à 3 minutes, plus préférentiellement allant de 0 à 2 minutes, encore plus préférentiellement égale à 2 minutes.

13. Procédé selon l'une quelconque des revendications 10 à 12, dans lequel lesdits premier et deuxième trempages par immersion dudit panier percé contenant l'échantillon consistent à immerger complètement lesdits dispositifs médicaux dans les solutions susvisées.

14. Procédé selon l'une quelconque des revendications 10 à 13, dans lequel une étape de rinçage, de préférence à l'eau, dudit échantillon est appliqué directement après ledit deuxième trempage, par immersion dudit panier percé contenant l'échantillon.

15. Utilisation du kit selon l'une quelconque des revendications 1 à 9, pour contrôler la qualité de nettoyage de dispositifs médicaux.

16. Utilisation du kit selon l'une quelconque des revendications 1 à 11, pour ajuster l'efficacité du nettoyage d'une composition détergente comprenant au moins une enzyme.

17. Utilisation d'une solution de coloration par trempage d'un échantillon prélevé aléatoirement parmi des dispositifs médicaux, ladite solution de coloration comprenant un colorant dans une phase de dilution, dans un procédé de contrôle de la qualité de nettoyage des dispositifs médicaux, ledit colorant dans ladite phase de dilution est compris dans le groupe des colorants constitué du bleu de Coomassie, et du noir Amide, ladite solution de coloration comprend un colorant à une concentration allant de 0.05% à 1%, ladite phase de dilution comprend au moins de l'acide citrique et/ou de l'acide lactique ; ladite phase de dilution est une phase de dilution compatible avec les exigences liées au milieu médical, duquel ledit échantillon provient, contenant un ou plusieurs dispositifs médicaux, éventuellement souillés de composés protéiques et/ou de matières organiques et/ou de biofilms présents sur lesdits dispositifs médicaux.

## Patentansprüche

1. Kit zur Kontrolle der Reinigungsqualität einer Probe, umfassend:
- eine Färbelösung durch Eintauchen der Probe, wobei die Färbelösung einen Farbstoff in einer Verdünnungsphase umfasst, und
- eine Indikatorlösung durch Eintauchen der Probe, wobei die Indikatorlösung die Verdünnungsphase umfasst,
**dadurch gekennzeichnet, dass** der Farbstoff in der Verdünnungsphase in der Gruppe der Farbstoffe enthalten ist, die aus Coomassie-Blau und Amid-Schwarz besteht, die Färbelösung einen Farbstoff in einer Konzentration im Bereich von 0,05 % bis 1 % umfasst, die Verdünnungsphase mindestens Zitronensäure und/oder Milchsäure umfasst; die Verdünnungsphase eine Verdünnungsphase ist, die mit den Anforderungen des Medizinbereichs, aus dem die Probe stammt, kompatibel ist, welche ein oder mehrere Medizinprodukte enthält, die möglicherweise mit Proteinverbindungen und/oder organischen Stoffen und/oder Biofilmen, die auf den Medizinprodukten vorhanden sind, kontaminiert sind.

2. Kit nach Anspruch 1, wobei die Färbelösung einen Farbstoff bevorzugt in einer Konzentration von 0,05% bis 0,5% und bevorzugter in einer Konzentration von 0,1% bis 0,2% umfasst.

3. Kit nach Anspruch 1 oder 2, wobei die Färbelösung einen pH-Wert zwischen 1 und 9, bevorzugt zwischen 1 und 7, bevorzugter zwischen 1 und 5, und besonders bevorzugt zwischen 1 und 3 oder zwischen 3 und 5 aufweist.

4. Kit nach einem der voranstehenden Ansprüche, wobei die Verdünnungsphase eine wässrige Lösung ist.

5. Kit nach einem der voranstehenden Ansprüche, wobei die Verdünnungsphase mindestens eine Säure und Wasser umfasst.

6. Kit nach einem der voranstehenden Ansprüche, wobei die Verdünnungsphase außerdem ein organisches Lösungsmittel umfasst.

7. Kit nach einem der voranstehenden Ansprüche, wobei die Verdünnungsphase mindestens eine Säure, ausgewählt aus der Gruppe bestehend aus Milchsäure, Zitronensäure, Essigsäure, Oxalsäure, Phosphorsäure, und gegebenenfalls mindestens ein organisches Lösungsmittel, ausgewählt aus der Gruppe bestehend aus Ethanol, Isopropanol, Glykolethern, Ether, Aceton, Propanol, Butanol, Petrolethern und deren Mischungen, umfasst.

8. Kit nach einem der voranstehenden Ansprüche, wobei die Verdünnungsphase mindestens ein Additiv und Wasser umfasst.

9. Kit nach einem der voranstehenden Ansprüche, wobei die Medizinprodukte aus der Gruppe bestehend aus zahnärztlichen Instrumenten, chirurgischen Instrumenten mit oder ohne Lumina, Endoskopen und sonstigen Medizinprodukten ausgewählt sind, wobei die Medizinprodukte von geringer Porosität und/oder geringer Rauheit sind und aus mindestens einem Material wie rostfreiem Stahl, Titan, Wolframkarbid, Chrommaterialien, Kunststoffen, Glas, Aluminium, eloxiertem Aluminium, Edelstahl, Kupfer, Messing, polyamidbasierten Kunststoffen, Polyethylen, PVC, POM, ABS, Acrylglas, Polyphenylsulfon, Polypropylen, Teflon (PTFE), Polycarbonaten, in ihren mit medizinischen Anwendungen kompatiblen Formen und Kombinationen davon, bestehen, und die zuvor einer Reinigung im Hinblick auf eine Sterilisation unterzogen wurden.

10. Verfahren zur Kontrolle der Reinigungsqualität von Medizinprodukten, bevorzugt unter Verwendung des Kits nach einem der voranstehenden Ansprüche, umfassend die folgenden Schritte:
- Bereitstellung einer zufällig aus Medizinprodukten ausgewählten Probe, die zuvor im Hinblick auf eine Sterilisation gereinigt oder im Hinblick auf eine Qualitätskontrolle gereinigt und sterilisiert wurden.
- Inkontaktbringen der Probe mit einer Färbelösung, die einen Farbstoff in einer Verdünnungsphase umfasst, die mit den Anforderungen des Medizinbereichs kompatibel ist,
- Kontrolle der Reinigungsqualität der Probe durch mögliche Identifizierung durch Färbung,
**dadurch gekennzeichnet, dass** der Farbstoff in der Verdünnungsphase in der Gruppe der Farbstoffe enthalten ist, die aus Coomassie-Blau und Amid-Schwarz besteht, die Probe ein oder mehrere zufällig ausgewählte Medizinprodukte mit geringer Porosität und/oder geringer Rauheit umfasst, und in einen durchlochten Korb gegeben wird, wobei das Inkontaktbringen durch ein erstes Eintauchen in ein Tauchbad des die Probe enthaltenden, durchlochten Korbes in eine Färbelösung und durch ein zweites Eintauchen nach dem Färben in ein Tauchbad des die Probe enthaltenden, durchlochten Korbes in eine die Verdünnungsphase umfassende Indikatorlösung erfolgt, und dass die Identifizierung durch Färbung eine Färbung zum Sichtbarmachen von Proteinverbindungen und/oder organischem Material und/oder Biofilm ist.

11. Verfahren nach Anspruch 10, wobei das Eintauchen in ein Tauchbad des durchlochten Korbes, der die Probe enthält, für eine Zeitdauer von weniger als 10 Minuten, bevorzugt weniger als 7 Minuten, bevorzugter zwischen 2 und 5 Minuten, und besonders bevorzugt gleich 5 Minuten durchgeführt wird.

12. Verfahren nach Anspruch 10 oder 11, wobei das zweite Eintauchen in ein Tauchbad des durchlochten Korbes, der die Probe enthält, für eine Zeitdauer von weniger als 5 Minuten, bevorzugt weniger als 3 Minuten, bevorzugter zwischen 0 und 2 Minuten, und besonders bevorzugt gleich 2 Minuten durchgeführt wird.

13. Verfahren nach einem der Ansprüche 10 bis 12, wobei das erste und das zweite Eintauchen in ein Tauchbad des durchlochten Korbes, der die Probe enthält, darin bestehen, die Medizinprodukte vollständig in die genannten Lösungen einzutauchen.

14. Verfahren nach einem der Ansprüche 10 bis 13, wobei ein Spülschritt, bevorzugt mit Wasser, der Probe direkt nach dem Eintauchen in ein Tauchbad des durchlochten Korbes, der die Probe enthält, durchgeführt wird.

15. Verwendung des Kits nach einem der Ansprüche 1 bis 9 zur Kontrolle der Reinigungsqualität von Medizinprodukten.

16. Verwendung des Kits nach einem der Ansprüche 1 bis 11 zur Anpassung der Reinigungswirkung einer Detergenszusammensetzung, die mindestens ein Enzym umfasst.

17. Verwendung einer Färbelösung durch Eintauchen einer zufällig aus Medizinprodukten ausgewählten Probe, wobei die Färbelösung einen Farbstoff in einer Verdünnungsphase umfasst, in einem Verfahren zur Kontrolle der Reinigungsqualität von Medizinprodukten, wobei der Farbstoff in der Verdünnungsphase in der Gruppe der Farbstoffe enthalten ist, die aus Coomassie-Blau und Amid-Schwarz besteht, wobei die Färbelösung einen Farbstoff in einer Konzentration von 0,05% bis 1% umfasst, wobei die Verdünnungsphase mindestens Zitronensäure und/oder Milchsäure umfasst; die Verdünnungsphase eine Verdünnungsphase ist, die mit den Anforderungen des Medizinbereichs, aus der die Probe stammt, kompatibel ist, welche ein oder mehrere Medizinprodukte enthält, die möglicherweise mit Proteinverbindungen und/oder organischen Stoffen und/oder Biofilmen, die auf den Medizinprodukten vorhanden sind, kontaminiert sind.

## Claims

1. Kit for controlling the quality of cleaning a sample, which comprises:
- a solution for colouring by soaking said sample, said colouring solution comprising a colourant in a dilution phase, and
- a solution for revelation by soaking said sample, said revelation solution comprising said dilution phase,
**characterised in that** said colourant in said dilution phase is comprised in the group of colourants consisting of Coomassie blue, Amido black, said colouring solution comprises a colourant at a concentration going from 0.05% to 1%, said dilution phase comprises at least citric acid and/or lactic acid; said dilution phase is a dilution phase compatible with the requirements linked to the medical sector, from which said sample comes, containing one or more medical devices, possibly contaminated by protein compounds and/or organic matters and/or biofilms present on said medical devices.

2. Kit according to claim 1, wherein said colouring solution comprises a colourant preferably at a concentration going from 0.05% to 0.5% and more preferably, at a concentration going from 0.1% to 0.2%.

3. Kit according to claim 1 or 2, wherein said colouring solution has a pH comprised between 1 and 9, preferably 1 and 7, more preferably between 1 and 5, even more particularly between 1 and 3 or between 3 and 5.

4. Kit according to any one of the preceding claims, wherein said dilution phase is an aqueous solution.

5. Kit according to any one of the preceding claims, wherein said dilution phase comprises at least one acid and water.

6. Kit according to any one of the preceding claims, wherein said dilution phase further comprises an organic solvent.

7. Kit according to any one of the preceding claims, wherein said dilution phase comprises at least one acid chosen from the group consisting of lactic acid, citric acid, acetic acid, oxalic acid, phosphoric acid, and possibly at least one organic solvent chosen from the group consisting of ethanol, isopropanol, glycol ethers, ether, acetone, propanol, butanol, petroleum ethers and their mixtures.

8. Kit according to any one of the preceding claims, wherein said dilution phase comprises at least one additive and water.

9. Kit according to any one of the preceding claims, wherein said medical devices are chosen from the group consisting of dentistry, surgical instruments with or without lumens, endoscopes and any other medical device, said medical devices being of low porosity and/or low roughness are formed of at least one material like stainless steel, titanium, tungsten carbide, chrome, plastic, glass, aluminium, anodised aluminium, stainless steel, copper, brass materials, synthetic polyamide-, polyethylene-, PVC-,. POM-, ABS-based materials, acrylic glass, polyphenylsulfone, polypropylene, Teflon (PTFE), polycarbonates, in their forms which are compatible with medical applications and their combinations and which have previously been subjected to cleaning in view of sterilisation.

10. Method for controlling the quality of cleaning of medical devices, preferably by using the kit according to any one of the preceding claims, comprising the following steps:
- making a sample randomly sampled from among medical devices available, previously cleaned in view of sterilisation, or previously cleaned and sterilised in view of quality control,
- putting said sample in contact through a colouring solution which comprises a colourant in a dilution phase which is compatible with the requirements linked to the medical sector,
- controlling the quality of cleaning the sample through a possible identification by colouring,
**characterised in that** said colourant in said dilution phase is comprised in the group of colourants consisting of Coomassie blue and Amido black, said sample comprises one or more medical devices of low porosity and/or low roughness chosen randomly and is placed in a basket with a hole, said putting into contact being carried out by a first soaking by immersion of said basket with a hole containing the sample in a colouring solution and through a second soaking after colouring, by immersion of said basket with a hole containing the sample in a revelation solution comprising said dilution phase and **in that** said identification by colouring in a colouring in the view of protein compounds and/or organic matters and/or biofilm.

11. Method according to claim 10, wherein said first soaking by immersion of said basket with a hole containing the sample is carried out during a time period less than 10 minutes, preferably less than 7 minutes, more preferably going from 2 to 5 minutes, even more preferably equal to 5 minutes.

12. Method according to claim 10 or 11, wherein said second soaking by immersion of said basket with a hole containing the sample is carried out during a time period less than 5 minutes, preferably less than 3 minutes, more preferably going from 0 to 2 minutes, even more preferably equal to 2 minutes.

13. Method according to any one of claims 10 to 12, wherein said first and second soakings by immersion of said basket with a hole containing the sample consist of fully immersing said medical devices in the abovementioned solutions.

14. Method according to any one of claims 10 to 13, wherein a step of rinsing, preferably with water, said sample is applied directly after said second soaking, by immersion of said basket with a hole containing the sample.

15. Use of the kit according to any one of claims 1 to 9, to control the quality of cleaning medical devices.

16. Use of the kit according to any one of claims 1 to 11, to adjust the effectiveness of the cleaning of a detergent composition comprising at least one enzyme.

17. Use of a solution of colouring by soaking a sample randomly sampled from among medical devices, said colouring solution comprising a colourant in a dilution phase, in a method for controlling the quality of cleaning medical devices, said colourant in said dilution phase is comprised in the group of colourants consisting of Coomassie blue and Amido black, said colouring solution comprises a colourant at a concentration going from 0.05% to 1%, said dilution phase comprises at least citric acid and/or lactic acid; said dilution phase is a dilution phase which is compatible with the requirements linked to the medical sector, from which said sample comes, containing one or more medical devices, possibly contaminated by protein compounds and/or organic matters and/or biofilms present on said medical devices.
